# EUROPEAN PATENT SPECIFICATION

(11) **EP 2 515 803 B1**
(45) Date of publication and mention of the grant of the patent: **30.08.2017**
(21) Application number: 10840066.4
(22) Date of filing: 21.12.2010
(51) Int. Cl.: A61F 2/82, A61F 2/00

(54) **INTRAVAGINAL INCONTINENCE DEVICE**
INTRAVAGINALE INKONTINENZVORRICHTUNG
DISPOSITIF INTRAVAGINAL CONTRE L'INCONTINENCE

(30) Priority: 23.12.2009 US 645800
(43) Date of publication of application: 31.10.2012
(73) Proprietor: First Quality Hygienic, Inc., Great Neck, NY 11021 (US)
(72) Inventor: HULL, JR., Raymond J., Hampton, NJ 08827 (US); JOHNSON, Bruce C., Whiting, NJ 08759 (US); MAVINKURVE, Pramod, Princeton, NJ 08540 (US); ROSENFELD, Leonard, Yardley, PA 19067 (US)
(74) Representative: Studio Torta S.p.A.
(86) International application number: PCT/US2010/061566
(87) International publication number: WO 2011/079124

(56) References cited:
- WO-A2-2009/044394
- US-A1- 2005 152 938
- US-A1- 2005 234 291
- US-A1- 2007 254 014
- US-A1- 2008 009 662
- US-A1- 2008 009 663
- US-A1- 2008 009 664
- US-A1- 2008 009 814
- US-A1- 2008 009 931
- US-A1- 2008 033 230
- US-A1- 2008 033 231
- US-A1- 2008 033 231
- US-B1- 6 418 930
- US-B1- 6 679 831
- LIU C ET AL: "Review of progress in shape-memory polymers", JOURNAL OF MATERIALS CHEMISTRY, ROYAL SOCIETY OF CHEMISTRY, GB, vol. 17, 1 January 2007 (2007-01-01), pages 1543-1558, XP009122354, ISSN: 0959-9428, DOI: 10.1039/B615954K

## Description

### Field of the Invention

The present invention relates to an intravaginal incontinence device comprising a polymeric resilient material. More specifically, this invention relates to a device that has a working portion having a variable equivalent diameter, a length suitable for insertion into a vagina and an anchoring mechanism for retention in the vagina, and is made from high modulus polymers having high tensile modulus properties along with high yield strain properties. Additionally, the polymers demonstrate resistance to creep and stress relaxation. The device is useful for reducing or preventing urinary incontinence.

### Description of the Prior Art

Stress urinary incontinence is a problem for many women. It is characterized by leakage of urine during a stressing event, such as a cough or a sneeze. Many devices have been designed to reduce or prevent stress urinary incontinence. Tutrone, Jr., US Pat. No. 5,603,685, teaches inflatable devices and a means to provide a device that is small for insertion into the vagina and enlarges to a required shape and pressure to reduce or prevent urinary incontinence. Zunker et al., US Pat. No. 6,090,098, teaches tamponlike devices, each made with a combination of absorbing and/or non-absorbing fibrous materials. Ulmsten et al., US Pat. No. 6,645,137, teaches a coil that expands in the vagina. Biswas, US Pat. No. 5,036,867, teaches a compressible resilient pessary. James, US Pat. No. 6,460,542, teaches a highly shaped rigid pessary. Many patents are drawn to stents that are sized and designed to keep arteries open.

Co-pending US Pat. App. No. 2008/0009664 (Bartning et al.) teaches urinary incontinence devices that may be made from shape memory polymers, which is defined as those materials that can be shaped into an initial shape, which can be subsequently formed into a stable second shape. The material is capable of substantially reverting to its initial shape upon exposure to an appropriate event. Among the shape memory materials disclosed in this publication are elastic or superelastic materials such as metal alloys such as Nitinol, phase segregated linear block co-polymers, biostable or bioabsorbable shape memory polymers (SMPs), etc. The SMPs can also be prepared from thermoplastic elastomers made from hydrophilic polymers. These elastomers typically have low modulus and may or may not be capable of significant stretching prior to breakage.

US2008/033231 A1 discloses a method for treating urinary incontinence includes the steps of inserting into the woman's vagina a first disposable device, removing the first disposable device, and inserting a second disposable device substantially identical to the first disposable device. The disposable devices have a working portion with opposed working surfaces to provide support to an associated urinary system and an anchoring portion to maintain the disposable device in place during use. The anchoring portion has at least one member extending beyond at least one end of the working portion

Despite the teaching of the prior art, there is a continuing need for a device suitable for insertion into a vagina and useful for reducing or preventing urinary incontinence. This device needs to be highly resilient.

### SUMMARY OF THE INVENTION

We have invented an intravaginal incontinence device that is useful for reducing or preventing urinary incontinence that can be produced reliably and economically. We have found that we can produce a highly resilient polymeric device that meets the needs of incontinent women.

The aforementioned results are obtained by an intravaginal incontinence device according to claim 1.

### BRIEF DESCRIPTION OF THE DRAWINGS

Fig. 1 is a front plan view of a device according to the present invention. Fig. 2 is a perspective view of an embodiment of the present invention. Fig. 3 is front plan view of the embodiment of Fig. 2.
Fig. 4 is a side elevation of the embodiment of Fig. 2.
Fig. 5 is a front plan view of the embodiment of Fig. 2 when restrained as packaged.
Fig. 6 is an anatomical cross-section of a woman using the embodiment of Fig. 2.

### DETAILED DESCRIPTION OF PREFERRED EMBODIMENTS

The challenge that has faced and continues to face developers of intravaginal incontinence devices is to provide an efficient, reliable polymeric device that can be delivered with a small cross-section for relatively easy insertion. At the same time the need exists to provide enough stiffness or force to push against vaginal walls to provide the necessary support to the bladder and other elements of the female urinary system. Materials used in previous disclosures include elastic and superelastic materials including metal alloys such as nitinol, phase segregated linear block co-polymers, and biostable or bioabsorbable shape memory polymers (SMPs), etc. However, these materials are either expensive or are not sufficiently resilient or stiff or elastic under desired operating conditions, or suffer from several of these drawbacks.

We have found that it is important to balance the resilience of the material that forms the device against its ability to be reduced in cross-section to provide easy insertion. Thus, it is useful to consider resilience and stiffness of the materials. If using a material for the device that is too stiff, it may be difficult to compress the device into a configuration that can be contained within the much smaller diameter of an applicator.

As used herein, the term "high modulus polymer" and variants thereof relate to polymers having high tensile modulus properties and high yield strain properties. The materials produced with these polymers are very tough with high impact strength.

As used herein the specification and the claims, the term "stent" and variants thereof relate to a device used to support a bodily orifice, cavity, vessel, and the like. The stent is resilient, flexible, and collapsible with memory. The stent may be any suitable form, including, but not limited to, scaffolding, a slotted tube or a wire form.

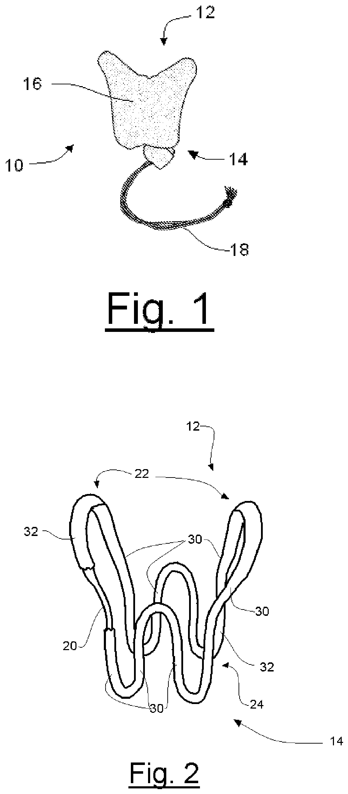

Suitable shapes of devices according to the present invention are taught in US Pat. App. No. 2008/0009664. Referring to Fig. 1 , there is shown a device 10 according to the present invention. The device 10 has an insertion end 12 and a withdrawal end 14. The device includes an outer flexible enclosure, such as a bag 16, substantially containing a resilient frame, e.g., a stent, and having a withdrawal element, such as string 18.

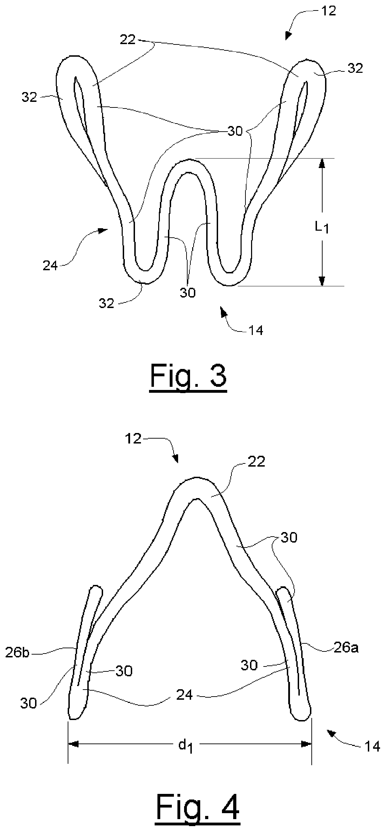

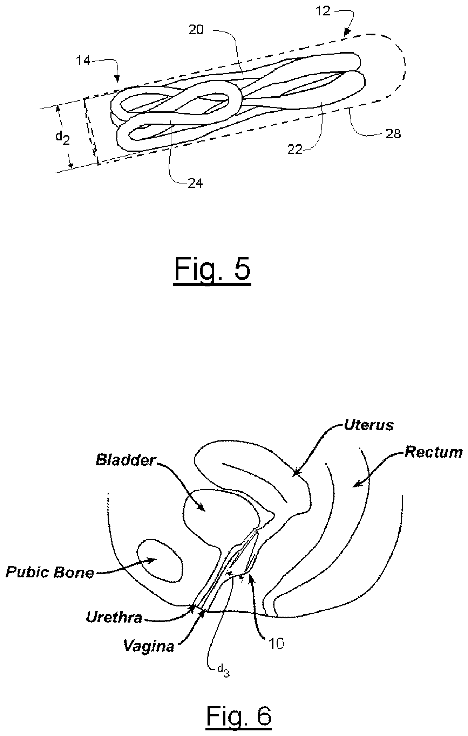

In one embodiment, the flexible enclosure 16 contains a resilient frame 20, such as shown in Figs. 2-5. The resilient frame 20 includes an anchoring portion 22 that is disposed proximate the insertion end 12, and a working portion 24 that is disposed proximate the withdrawal end 14. The working portion 24 has faces 26a and 26b. Working portion 24 has an initial equivalent diameter di ranging from 20 mm to 170 mm and a length l_i ranging from 15 mm to 60 mm. Where the working portion is non-cylindrical, the equivalent diameter is the maximum distance in millimeters between opposed faces. As seen in Fig. 5, working portion 24 has an insertion equivalent diameter 02 (in an applicator 28 or other device for insertion) ranging from 5 mm to 20 mm. As seen in Fig. 6, working portion 24 has a use equivalent diameter (in the vagina) d3 ranging from 5 mm to 40 mm.

Working portion 24 includes a high modulus polymeric structural material that compresses and recovers with sufficient force to provide the desired effect. Such high modulus polymers have an elongation at yield of at least 3% and an elastic modulus of at least 2 Gpa. A representative, non-limiting list of suitable high modulus polymers includes thermoplastics, thermosets, fiber-reinforced polymers, polyetherimide, polyetheretherketone, polycarbonate, co-polymers, specialized and/or modified plastics, filled plastics, and the like, that can provide these high modulus properties. Particularly preferred high modulus polymers include polyetherimides and polyetheretherketones.

As used herein the specification and the claims, the term "stent" and variants thereof relate to a device used to support a bodily orifice, cavity, vessel, and the like. The stent is resilient, flexible, and collapsible with memory. The stent may be any suitable form, including, but not limited to, scaffolding, a slotted tube or a wire form.

Suitable shapes of devices according to the present invention are taught in US Pat. App. No. 2008/0009664, the disclosure of which is hereby incorporated by reference in its entirety. Referring to Fig. 1, there is shown a device 10 according to the present invention. The device 10 has an insertion end 12 and a withdrawal end 14. The device includes an outer flexible enclosure, such as a bag 16, substantially containing a resilient frame, e.g., a stent, and having a withdrawal element, such as string 18.

In one embodiment, the flexible enclosure 16 contains a resilient frame 20, such as shown in Figs. 2-5. The resilient frame 20 includes an anchoring portion 22 that is disposed proximate the insertion end 12, and a working portion 24 that is disposed proximate the withdrawal end 14. The working portion 24 has faces 26a and 26b. Working portion 24 has an initial equivalent diameter d₁ ranging from 20 mm to 170 mm and a length L₁ ranging from 15 mm to 60 mm. Where the working portion is non-cylindrical, the equivalent diameter is the maximum distance in millimeters between opposed faces. As seen in Fig. 5, working portion 24 has an insertion equivalent diameter d₂ (in an applicator 28 or other device for insertion) ranging from 5 mm to 20 mm. As seen in Fig. 6, working portion 24 has a use equivalent diameter (in the vagina) d₃ ranging from 5 mm to 40 mm.

Working portion 24 includes a high modulus polymeric structural material that compresses and recovers with sufficient force to provide the desired effect. Such high modulus polymers have an elongation at yield of at least 3% and an elastic modulus of at least 2 Gpa. A representative, non-limiting list of suitable high modulus polymers includes thermoplastics, thermosets, fiber-reinforced polymers, polyetherimide, polyetheretherketone, polycarbonate, co-polymers, specialized and/or modified plastics, filled plastics, and the like, that can provide these high modulus properties. Particularly preferred high modulus polymers include polyetherimides and polyetheretherketones.

Again, high modulus polymers have high tensile modulus properties and high yield strain properties. Preferably, polymer has a tensile modulus, or elastic modulus, of at least about 2 Gigapascal (Gpa). In addition, it is preferred that the polymer has a high yield strain or an elongation at yield of at least about 3%, more preferably an elongation at yield of at least about 4.5% and most preferably an elongation at yield of at least about 5%. It is also preferred that the high modulus polymer is resistant to creep and stress relaxation.

In one embodiment, the working portion 24 comprises a plurality of connected elongate elements, such as struts 30. One or more of these elongate elements 30 may directly or indirectly tie the working portion 24 to the anchoring portion 22. The longitudinal projection of the working portion elongate elements defines the working portion length L₁. The working pressure exerted by the working portion 24 is determined by the particular high modulus material selected and by the dimensions of the elongate elements. Thicker elongate elements and/or shorter elongate elements generally provide greater working pressures. In addition, the angle between the elongate elements also influences the working pressure.

The elongate elements 30 have a diameter much less than that of the working and/or anchoring portions. Preferably, the elongate elements have a diameter of less than about 5 mm, more preferably between about 1 mm and about 4 mm, even more preferably, between about 1.5 mm and about 3 mm. If the diameter of the elongate element is too large, the device may become too stiff and too large to appropriately compress the device for easy insertion. If the diameter is too small, the device may not be able to provide sufficient force to support the urinary system.

For some applications, the working portion exerts an expansion force, as described below, of from about 2 to about 8 Newtons ("N") in the working state, preferably about 2 to about 6 N, and more preferably about 3 to about 6 N. Frame 20 also has an anchoring portion 22. Anchoring portion 22 is shaped suitable to keep the device 10 in place while in use. Suitable shapes include, but are not limited to, a basket handle, a dog bone, wings, and rabbit ears. The anchoring portion 22 may be made of the same material as the working portion 24 or they may be made of different materials. The working portion 24 and anchoring portion 22 may be made as a uni-body construction, or may be made separately and joined by attachment means. The frames 20 may be treated to provide improved biocompatibility. The frame 20 may be partially or completely covered, e.g., by placing inside tubing, by coating, molding, etc., to improve biocompatibility and/or comfort. The embodiment of Figs. 2-5 shows a frame 20 covered by an outer layer 32 of material (with the outer layer 32 broken away in Fig. 2 to show the frame 20).

Devices according to the present invention may be useful for treating or preventing urinary incontinence. For this application, the device is sized to fit comfortably in the vagina. The devices described below may have working portions with initial equivalent diameters of from about 20 to about 170 mm. Preferably, the working portion has a working portion that may have an initial equivalent diameter ranging from about 20 to about 170 mm, preferably about 20 to about 45 mm, or more preferably about 30 mm; an insertion equivalent diameter ranging from about 5 to about 25 mm, preferably about 10 to about 20 mm, or more preferably about 18 mm; a use equivalent diameter ranging from about 10 20 to about 40 mm, preferably about 25 to about 30 mm, or more preferably about 25 mm; and a length ranging from about 20 to about 60 mm, preferably about 20 to about 30 mm, or more preferably about 25 mm.

The anchoring portion extends beyond the working portion in a direction away from the vaginal opening and may have an initial equivalent diameter ranging from about 30 to about 200 mm, preferably about 40 to about 60 mm, or more preferably about 50 mm; an insertion equivalent diameter ranging from about 10 to about 25 mm, preferably about 10 to about 20 mm, or more preferably about 18 mm; a use equivalent diameter ranging from about 20 to about 100 mm, preferably about 40 to about 60 mm, or more preferably about 50 mm; and a length ranging from about 10 to about 50 mm, preferably about 20 to about 40 mm, or more preferably about 30 mm.

The anchoring portion of the device has a length and width in the insertion state, the working state, and upon removal. The insertion state length may range from about 25 to about 40 mm, for example about 30 mm. The insertion state width may range from about 10 to about 20 mm, for example about 18 mm. The working state length at rest and during a cough may range from about 25 to about 40 mm, for example about 30 mm. The working state width at rest and during a cough may range from about 15 to about 35 mm, for example about 30 mm.

As shown in Fig. 1, the frame 20 may be enclosed in a flexible enclosure 16, such as a bag, that may reduce friction during deployment, shield the frame 20 from view (to be aesthetically pleasing), help control the device 10 during insertion and removal, help the device to stay in place, and/or create more contact area for applying pressure to the bladder neck. The flexible enclosure 16 may also provide increased friction against the vaginal epithelium to reduce the likelihood of undesired movement during use, e.g., becoming skewed. Any medically appropriate materials may be used to form the flexible enclosure, and depending upon the desired end-use, it may be opaque, light, and/or breathable. Useful flexible enclosure materials include those used in the manufacture of tampons, such as nonwoven fabrics and plastic film, including apertured films. The flexible enclosure itself may also be apertured. In one embodiment, the frame was placed in a heat-sealed bag made of non-woven polypropylene material used in intravaginal tampon covers. The covered frame can be easily removed by the addition of a withdrawal element 18, such as an ordinary tampon string.

The withdrawal element 18 may be crisscrossed between the elongate elements 30 of the frame 20 to create a "cinch sac" mechanism. Any string or cord known in the sanitary protection art may be useful for this purpose. As the strings are pulled during removal, the struts are gathered together to create a smaller diameter device during removal. Cinching the device at its base may make removal of the device more comfortable and easier as it makes the diameter of the device smaller and the shape conducive to remove easily.

As shown in Fig. 5, the device 10 may be contained within an applicator 28 similar to those known for use in delivering tampons and suppositories. The applicator may be a push-type applicator or a retractable applicator. A collar may be added to control the depth of insertion.

The resilient frame 20 can be made by any known polymer manufacturing methods. Preferably, the frame is injection molded, and the high modulus polymer can be selected for processability in these systems. The resilient frame 20 can then be further coated and or enclosed in a bag by known means.

### Examples

The following examples are illustrative of devices according to the present invention. The claims should not be construed to be limited to the details thereof.

### Expansion Force Test

The outward force that the polymeric frame exerts at various compression states was measured using an Instron Universal Testing machine (Model 1122, Instron Corp., Canton, MA) in a room at 23° ± 2° C. The Universal Testing machine was equipped with two opposing and rigid horizontal plates. One plate was attached to the crosshead. The other plate faced the first one and was attached to the upper fixed surface. The lower plate moved upward with the crosshead to compress the sample. Both plates were otherwise rigid and made of aluminum or steel. The upper plate was affixed to a load cell capable of measuring forces compression forces between 0 and 30 Newtons ("N").

To run the test, the plates were brought to an initial spacing of 37 mm. Either before or after aging, each device was placed with the two sides of the working section placed against the plates. Where the working portion of the device is cylindrical, opposing sides of the cylinder contact the plates. For non-cylindrical devices, the front and rear faces contact the plates. Via crosshead movement, the plates were brought together at a rate of 25.4 mm / minute until they are 10 mm apart. Force against the load cell is recorded automatically as the plates moved together. The expansion force at 20 mm spacing is the recorded measurement. For the initial time (no storage) samples, the devices were not placed within an applicator. For the 5 minute storage time point, each device was tested after being contained in a 15.6 mm inner diameter applicator for 5 minutes at room temperature. For the final time point, each device was placed within a 15.6 mm inner diameter applicator and stored for three weeks at 40° C. After the pre-determined time, the devices were expelled and allowed to come to room temperature without constraint for 15 to 30 minutes. For each time point, n=3. The results are shown below in Table 1.

Note: The applicator had an inner diameter of 15.6 mm and an outer diameter of 18 mm. It was injection molded of polyethylene. These dimensions and materials were chosen to minimize deformation during storage.

**Table 1**

| Sample No. | Commercial Name, Code, Supplier | Generic name | Expansion Force (N) @ 20 mm t = 0 (No storage) | Expansion Force (N) @ 20 mm t = 5 min (storage @ 23° C) | Expansion Force (N) @ 20 mm t = 3 weeks (storage @ 40° C) |
|---|---|---|---|---|---|
| 1 | Ultem®1010, SABIC Americas, Inc (Houston, TX) | Polyetherimide | 8.24 | 8.38 | 4.77 |
| 2 | Victrex® 450G Victrex USA Inc. (West Consho-hocken, PA) | Polyetheretherketone | 7.06 | 7.53 | 4.89 |
| 3 | Profax SR549 Basell North America, Inc. Wilmington, DE | Polypropylene | 2.48 | 1.82 | 0.13 |
| 4 | Cycolac MG47 SABIC Americas, Inc (Houston, TX) | Acrylonitrile butadiene styrene | 5.87 | 4.34 | 0.01 |
| 5 | Petrothene NA831 Basell North America, Inc. Wilmington, DE | Low Density Polyethylene | 0.41 | 0.36 | 0.04 |
| 6 | Calibre 2061 Dow, Inc. Midland, MI | Polycarbonate | 5.66 | 5.16 | 1.45 |

As shown above in Table 1, some of the polymers exhibited a significant lack of Expansion Force, severe decrease over time in expansion capability compared to the initial time. After 3 weeks storage at 40° C, the devices of only two materials appeared to have maintained expansion properties of at least 2 N. This indicates that when devices are made from the materials having low creep values, the device will maintain its ability to expand and provide sufficient pressure once in place in the vagina.

Preferred materials, those that maintain expansion properties of greater than 1 N include polycarbonate, polyetheretherketone, and polyetherimide. At the same time, it is clear that the polycarbonate exhibits a significant drop-off in expansion capability after prolonged storage and that polyetherimide and polyetheretherketone have more desirable properties.

In order to determine generic material properties that are indicative of the ability of a given material to provide sufficient expansion capability after prolonged storage, additional testing of sample material bars of the materials used to make the devices in Table 1 was conducted. Material properties tested included elastic modulus, yield strain and stress relaxation.

Elastic modulus and yield strain were determined by ASTM method D638-2008, as published on February 6, 2009. An ASTM type I tensile specimen with a thickness of about 3 mm was used. Actual thicknesses were measured and included in the calculations. Sample were conditioned for 40 hours at 23° C and 50% relative humidity ("RH") before testing and tested at 23° C and 49% RH. Cross head speed was 5 mm/minute. Elastic modulus (in Giga Pascals, GPa) was taken from the linear regression slope of the engineering stress vs engineering strain data points between 0.05 and 0.25% strain. Yield strain was calculated as the strain at peak stress on the engineering stress vs enginering strain curve. Non contact extensometers were used for all strain measurements.

Stress Relaxation Testing was done on an Instron Universal testing machine (Model 5582 electromechanical) with a temperature controlled environmental chamber. ASTM Type I tensile bars (approx 3 mm thickness) were initially pulled at a rate of 5 mm /minute with a noncontact extensometer measuring strain in the uniform central section of the bar. The data was used to identify the initial load needed to obtain a 3% strain in the uniform central region. New samples were then loaded to the target load while the extensometer measured strain. Over a 50 hour period the crosshead was automatically adjusted to maintain 3% strain in the center section while the applied load was monitored and stress was calculated. Stress was calculated as the applied load divided by the initial cross sectional area of the sample in the uniform central section (Mega Pascals, MPa) to determine the "Aged Stress." Finally, the "Stress Ratio" is expressed as a percentage of the Aged Stress to the Initial Stress.

The results of the testing of these materials are shown in Table 2, below.

| Sample No. | Commercial Name | Elastic Modulus (Gpa) | Elongation @ Yield (%) | Stress Relaxation (Stress @ 3% Strain) | | |
|---|---|---|---|---|---|---|
| | | | | Initial Stress (t = 0) (MPa) | Aged Stress (t = 50 hrs @ 40° C) (MPa) | Stress Ratio |
| 1 | Ultem® 1010 | 3.6 | 6.95 | 91.08 | 50.57 | 0.56 |
| 2 | Victrex® 450G | 4.4 | 5.17 | 81.60 | 59.00 | 0.72 |
| 3 | Profax SR549 | 1.3 | 12.09 | 19.40 | 6.40 | 0.33 |
| 4 | Cycolac MG47 | 2.4 | 2.27 | 35.20 | 11.30 | 0.32 |
| 5 | Petrothene NA831 | 0.3 | 100.26 | 3.40 | 1.70 | 0.50 |
| 6 | Calibre 2061 | 2.4 | 6.01 | 52.90 | 32.20 | 0.61 |

As shown by Tables 1 and 2, not all polymers have the appropriate elastic modulus, elongation and stress relaxation properties. Combinations of these properties are indicative of materials that provide appropriate modulus to produce a highly resilient polymeric device that meets the needs of incontinent women.

Therefore, a comparison of the materials' properties reported in the tables suggests that those materials that have relatively limited stress relaxation, those materials that have a Relative Strength of greater than 50% may be used in the present invention.

In summary, useful materials have an elastic modulus of at least about 2 GPa, preferably at least about 3 GPa. The elongation at yield is at least about 2%, preferably at least about 3%, and most preferably at least about 5%. The Initial Stress (t=0 hrs) @ 3% Strain is at least about 30 MPa, preferably at least about 50 MPa, and most preferably at least about 80 MPa. The Aged Stress (t=50 hrs @ 40° C) @ 3% Strain is at least about 30 MPa, preferably at least about 35 MPa, and more preferably at least about 50 MPa. Finally, the Stress Ratio is at least about 0.5, preferably at least about 0.7, and more preferably at least about 0.8.

In one preferred embodiment, the device comprises a material having an elastic modulus of at least about 2 GPa and an Initial Stress @ 3% strain of at least about 30 MPa. More preferred, the device has an elongation at yield of at least about 3%.

In another preferred embodiment, the device comprises a material having an elastic modulus of at least about 3 GPa and a Stress Ratio of at least about 0.5. More preferred, the device has an elongation at yield of at least about 3% and an Aged Stress (t=50 hrs @ 40° C) @ 3% Strain of at least about 35 MPa, and more preferred at least about 50 MPa.

In another preferred embodiment, the device comprises a material having an elastic modulus of at least about 2 GPa and a Stress Ratio of at least about 0.7. More preferred, the device has an elongation at yield of at least about 3% and an Aged Stress (t=50 hrs @ 40° C) @ 3% Strain of at least about at least about 35 MPa.

In another preferred embodiment, the device comprises a material having an elastic modulus of at least about 2 GPa and Aged Stress (t=50 hrs @ 40° C) @ 3% Strain of at least about 30 MPa, and more preferred at least about 35 MPa, and most preferably at least about 50 MPa. In addition, the material of this embodiment has an elongation at yield of at least about 3%.

Since many variations and embodiments of the invention can be made without departing from its spirit and scope, the invention resides in the claims hereinafter appended

## Claims

1. An intravaginal urinary incontinence device (10) having an insertion end (12) and an opposed
withdrawal end (14), the device comprising a resilient frame (20) comprising:
a working portion (24) disposed proximate the withdrawal end (14) comprising a resilient structure having a plurality of connected elongate elements (30) arranged and configured to define opposed working surfaces for providing support to an associated urinary system;
**characterized by** comprising an anchoring portion disposed proximate the insertion end (12) and extending beyond the working portion (24) comprising a plurality of connected elongate elements (30) arranged and configured to expand laterally within a user's vagina wherein the elongate elements (30) comprise a high modulus polymer selected from the group consisting of polyetherimides and/or polyetheretherketones, and combinations thereof, and having an elastic modulus of at least 2 GPa and an Initial Stress @ 3% Strain of at least about 30 MPa.

2. The device according to claim 1 wherein the high modulus polymer has an elongation at yield of at least 3%.

3. The device according to claim 1 wherein the elastic modulus is at least 3 GPa and a Stress Ratio of at least about 0.5, wherein the Stress Ratio is expressed as a percentage of the Aged Stress to the Initial Stress.

4. The device according to claim 1 wherein the working portion (24) comprises a plurality of connected generally longitudinally-oriented elongate elements (30).

5. The device according to claim 4 wherein the working portion (24) has a length ranging from about 10 to about 50 mm.

6. The device according to claim 1 further comprising an outer layer (32) of material at least partially covering an outer surface of the frame (20).

7. The device according to claim 1 further comprising a flexible enclosure substantially containing the frame (20).

8. The device according to claim 1 wherein the working portion (24) has an initial equivalent diameter ranging from about 20 to about 60 mm and an insertion equivalent diameter ranging from about 10 to about 25 mm, and a length ranging from about 10 to about 50 mm.

9. The device according to claim 8 wherein the anchoring portion has an initial equivalent diameter that is greater than the initial equivalent diameter of the working portion (24).

## Patentansprüche

1. Intravaginale Harninkontinenzvorrichtung (10) mit einem Einführende (12) und einem gegenüberliegenden Entnahmeende (14),
wobei die Vorrichtung einen elastischen Rahmen (20) umfasst, der Folgendes umfasst:
einen Arbeitsabschnitt (24), der in Nähe des Entnahmeendes (14) angeordnet ist und eine elastische Struktur umfasst, die eine Vielzahl verbundener länglicher Elemente (30) aufweist, die angebracht und ausgestaltet sind, sich gegenüberliegende Arbeitsflächen zum Unterstützen eines zugehörigen Harnsystems zu bestimmen;
**dadurch gekennzeichnet, dass** die Vorrichtung einen Verankerungsabschnitt, der proximal zum Einführende (12) angeordnet ist, umfasst, und sich über den Arbeitsabschnitt (24) hinaus erstreckt, der eine Vielzahl verbundener länglicher Elemente (30) umfasst, die angeordnet und ausgestaltet sind, sich in der Vagina einer Benutzerin seitlich auszudehnen, wobei die länglichen Elemente (30) ein Hochmodul-Polymer aus der Gruppe bestehend aus Polyetherimiden und/oder Polyetheretherketonen, oder Verbindungen daraus umfassen, und einen Elastizitätsmodul von mindestens 2 GPa und bei 3 % Dehnung eine Anfangsspannung von mindestens etwas 30 MPa aufweisen.

2. Vorrichtung nach Anspruch 1, wobei das Hochmodul-Polymer eine Dehnung bei Streckgrenze von mindestens 3 % aufweist.

3. Vorrichtung nach Anspruch 1, wobei der Elastizitätsmodul bei mindestens 3 GPa und ein Spannungsverhältnis bei mindestens etwa 0,5 liegt, wobei das Spannungsverhältnis prozentual als Verhältnis der mechanischen Spannung zu der Anfangsspannung ausgedrückt wird.

4. Vorrichtung nach Anspruch 1, wobei der Arbeitsabschnitt (24) eine Vielzahl verbundener, im Allgemeinen in Längsrichtung ausgerichteter länglicher Elemente (30) umfasst.

5. Vorrichtung nach Anspruch 4, wobei der Arbeitsabschnitt (24) eine Länge im Bereich von etwa 10 bis etwa 50 mm aufweist.

6. Vorrichtung nach Anspruch 1, die überdies eine äußere Materialschicht (32) umfasst, die zumindest teilweise eine Außenfläche des Rahmens (20) bedeckt.

7. Vorrichtung nach Anspruch 1, die überdies ein flexibles Gehäuse umfasst, das den Rahmen (20) im Wesentlichen enthält.

8. Vorrichtung nach Anspruch 1, wobei der Arbeitsabschnitt (24) einen anfänglichen Äquivalentdurchmesser im Bereich von etwa 20 bis etwa 60 mm, einen Einsetz-Äquivalentdurchmesser im Bereich von etwa 10 bis etwa 50 mm, und eine Länge im Bereich von etwa 10 bis etwa 50 mm aufweist.

9. Vorrichtung nach Anspruch 8, wobei der Verankerungsabschnitt einen anfänglichen Äquivalentdurchmesser aufweist, der größer ist als der Ausgangs-Äquivalentdurchmesser des Arbeitsabschnitts (24).

## Revendications

1. Dispositif intravaginal contre l'incontinence urinaire (10) présentant une extrémité d'insertion (12) et une extrémité de retrait opposée (14), le dispositif comprenant une armature élastique (20) comprenant:
une partie de travail (24) disposée à proximité de l'extrémité de retrait (14) et comprenant une structure élastique présentant une pluralité d'éléments allongés connectés (30) agencés et configurés de manière à définir des surfaces de travail opposées destinées à fournir un support à un système urinaire associé;
**caractérisé en ce qu'**il comprend une partie d'ancrage disposée à proximité de l'extrémité d'insertion (12) et s'étendant au-delà de la partie de travail (24) qui comprend une pluralité d'éléments allongés connectés (30) agencés et configurés de manière à se déployer latéralement à l'intérieur du vagin d'une utilisatrice, dans lequel les éléments allongés (30) comprennent un polymère à haut module d'élasticité sélectionné dans le groupe comprenant les polyétherimides et/ou les polyétheréthercétones, ainsi que des combinaisons de ceux-ci, et présentant un module d'élasticité d'au moins 2 GPa et une contrainte initiale à 3 % de déformation d'au moins environ 30 MPa.

2. Dispositif selon la revendication 1, dans lequel le polymère à haut module d'élasticité présente un allongement d'au moins 3 % dans la limite d'élasticité.

3. Dispositif selon la revendication 1, dans lequel le module d'élasticité est d'au moins 3 GPa, et un rapport de contraintes est d'au moins environ 0,5, dans lequel le rapport de contraintes est exprimé comme un pourcentage de la contrainte après usage par rapport à la contrainte initiale.

4. Dispositif selon la revendication 1, dans lequel la partie de travail (24) comprend une pluralité d'élément allongés connectés orientés de façon essentiellement longitudinale (30).

5. Dispositif selon la revendication 4, dans lequel la partie de travail (24) présente une longueur qui est comprise dans la gamme d'environ 10 mm à environ 50 mm.

6. Dispositif selon la revendication 1, comprenant en outre une couche extérieure (32) de matière qui couvre au moins partiellement une surface extérieure de l'armature (20) .

7. Dispositif selon la revendication 1, comprenant en outre une enveloppe souple qui contient sensiblement l'armature (20) .

8. Dispositif selon la revendication 1, dans lequel la partie de travail (24) présente un diamètre initial équivalent qui est compris dans la gamme d'environ 20 mm à environ 60 mm, et un diamètre d'insertion équivalent qui est compris dans la gamme d'environ 10 mm à environ 25 mm, et une longueur comprise dans la gamme d'environ 10 mm à environ 50 mm.

9. Dispositif selon la revendication 8, dans lequel la partie d'ancrage présente un diamètre initial équivalent qui est plus grand que le diamètre initial équivalent de la partie de travail (24).
